# EUROPEAN PATENT SPECIFICATION

(11) **EP 2 939 534 B1**
(45) Date of publication and mention of the grant of the patent: **25.01.2017**
(21) Application number: 14001527.2
(22) Date of filing: 30.04.2014
(51) Int. Cl.: A01K 67/027, A61K 36/77

(54) **Animal model for Parkes Weber syndrome**
Tiermodell für Parkes Weber Syndrom
Modèle animal pour le syndrome Parkes Weber

(43) Date of publication of application: 04.11.2015
(73) Proprietor: Warszawski Uniwersytet Medyczny, 02-091 Warszawa (PL); INSTYTUT FARMACEUTYCZNY, 01-793 Warszawa (PL)
(72) Inventor: Koziak, Katarzyna, 02-708 Warszawa (PL); Bojakowski, Krzysztof, 02-480 Warszawa (PL); Kowalewska, Magdalena, 03-937 Warszawa (PL); Maciejko, Dorota, 02-515 Warszawa (PL); Zegrocka-Stendel, Oliwia, 05-092 Lomianki (PL); Grabowska, Iwona, 11-300 Biskupiec (PL); Surowiecka, Agnieszka, 04-859 Warszawa (PL)
(74) Representative: Lazewski, Marek

(56) References cited:
- P. E. BURROWS ET AL: "Lymphatic abnormalities are associated with RASA1 gene mutations in mouse and man", PROCEEDINGS OF THE NATIONAL ACADEMY OF SCIENCES, vol. 110, no. 21, 21 May 2013 (2013-05-21) , pages 8621-8626, XP055145461, ISSN: 0027-8424, DOI: 10.1073/pnas.1222722110
- PHILIP E. LAPINSKI ET AL: "RASA1 maintains the lymphatic vasculature in a quiescent functional state in mice", JOURNAL OF CLINICAL INVESTIGATION, vol. 122, no. 2, February 2012 (2012-02), pages 733-747, XP055145449, ISSN: 0021-9738, DOI: 10.1172/JCI46116
- BOON L M ET AL: "RASA1: variable phenotype with capillary and arteriovenous malformations", CURRENT OPINION IN GENETICS & DEVELOPMENT, CURRENT BIOLOGY LTD, XX, vol. 15, no. 3, June 2005 (2005-06), pages 265-269, XP027675494, ISSN: 0959-437X [retrieved on 2005-06-01]
- Z Qian ET AL: "A simplified arteriovenous malformation model in sheep: feasibility study", AJNR. American journal of neuroradiology, May 1999 (1999-05), pages 765-770, XP055145630, UNITED STATES Retrieved from the Internet: URL:http://www.ncbi.nlm.nih.gov/pubmed/103 69342 [retrieved on 2014-10-10]

## Description

### FIELD OF THE INVENTION

The present invention relates to an animal model for Parkes Weber syndrome.

### BACKGROUND OF THE INVENTION

### Parkes Weber syndrome

The Parkes Weber (PW) syndrome, described in 1907, is characterized by varicose veins, enlarged limbs and presence of arteriovenous fistulas (AVF). The existence of AVF distinguishes PW from the Klippel-Trenaunay syndrome, known also as the *capillary-lymphatic-venous malformation*, CLVM. The symptoms of PW are congenital and present at birth. Vascular anomalies affect multiple limbs, less often trunk. Capillary malformations, forming geographic patterns, are commonly located on lateral side of the limb, buttocks or trunk. Additional laterally located veins appear in older age, which may be a consequence of vein anomalies and insufficiency of the deep venous system. Vein malformations in PW probably result from mutations in the *RASA1* gene, which affects normal development of vascular system. In 50 per cent of cases the hypoplasia of lymphatic system is also present and is manifested by lymphatic edema and isolated *lymphatic macrocitosis.* The overgrowth of a limb is present at birth, whereas, the axial overgrowth can enlarge while growing. In some cases the lower extremity can be shortened and contralateral hand or foot can be enlarged due to high adipose tissue content, in some cases without skin spots related to capillary malformations. When the pelvis is affected, often as a result of malformations in lower extremity, PW can be asymptomatic. The incidence of thromboembolism is reported in 20-45 per cent of cases, while pulmonary embolism, resulting from massive arteriovenous leak, is present in 4-25 per cent of the patients. As a consequence of arteriovenous leak, PW can lead to cardiac system failure or to limb ischemia.

The treatment of patients with PW is mainly symptomatic. Compression therapy (bandaging and stockings) and massages are used to reduce symptoms of chronic venous insufficiency and lymphatic edema. When recommended, intravascular and surgical procedures are performed. Surgical treatment is difficult and sometimes requires several intravascular procedures, such as embolization, sclerotherapy or classic open operations - arteriovenous fistula ligation. In severe cases of ischemic extremities, amputation is the only therapy.

To date, there has been no animal model reproducing complex manifestations of PW syndrome that could be used in research on etiology, treatment and prevention of the disease. Furthermore, the only attempt at creating an animal model for PW syndrome that has been reported so far represents the creation of Rasa1-deficient mice (Burrows PE at al. (2013) Lymphatic abnormalities are associated with RASA1 gene-mutations in mouse and man, Proc Natl Acad Sci USA. 2013 May 21;110(21):8621-6). Such animals develop lymphatic malformations which are also observed in PW syndrome patients, but other abnormalities characteristic for the disease are not present. Consequently, medical studies on PW syndrome have remained a challenge and in fact may only be performed with participation of human patients suffering from PW syndrome.

Therefore, there is an ever-existing need to provide an animal model of PW syndrome for the purpose of studying and evaluating PW syndrome, which allows for the testing of therapeutic agents for preventing, treating, delaying and ameliorating PW syndrome, as well as for testing materials for vascular therapy. The desired model should reflect the pathophysiological characteristics of PW syndrome and be an effective research tool in pharmacological studies, i.e. should allow the attempts of pharmacological approach to PW syndrome and the assessment of potential effect, such as prevention of limb overgrowth manifested in bone elongation and muscle hypertrophy.

### SUMMARY OF THE INVENTION

The invention in its broadest sense is as defined in the independent claims.

The present invention relates to the following:
1) A non-human animal mammalian model for PW syndrome, wherein an animal has an arteriovenous fistula and a venous ligature in at least one of the limbs, wherein the ligature narrows the vein lumen, and does not shut it off completely, the arteriovenous fistula is localised distally from the ligature, and there is no branch (collateral vein) between the ligature and the fistula.
2) A method for preparing a non-human animal mammalian model for PW syndrome as recited in the claims.
3) A method for studying PW syndrome, wherein the animal model of (1) is used.
4) Use of the animal model of (1) for studying PW syndrome.
5) Use of the animal model of (1) for screening a material for use in vascular therapy for PW syndrome.
6) A method of screening preventive and therapeutic agent for PW syndrome, wherein a test substance is administered to the animal model of (1).

### BRIEF DESCRIPTION OF THE DRAWINGS

Fig. 1 shows the results of measurements of venous pressure in operated and non-operated limbs.
Fig 2 shows results of measurements of thigh circumference of operated and non-operated limb.
Fig. 3a and 3b show results of measurements of *EDL* muscle weight and *soleus* muscle weight.
Figure 4 shows change in femoral bone length.
Figure 5 shows results of measurements of morphometric analysis in lower limb veins.
Figure 6 shows evaluation of the number, circumference and area of muscle fibers and estimation of connective tissue area in *EDL* muscle.
Figure 7 shows evaluation of the number, circumference and area of muscle fibers and estimation of connective tissue area in *soleus* muscle.
Fig. 8 shows the change in femoral bone length following β-escin treatment.

### DETAILED DESCRIPTION OF THE INVENTION

The present invention provides a non-human animal mammalian model for PW syndrome in which the surgical intervention performed on the animal causes PW syndrome with its characteristic symptoms.

The inventors of the present invention surprisingly observed that a specific arteriovenous fistula in combination with a specific venous ligature in at least one of the limbs causes PW syndrome. The ligature should only narrow the vein lumen (i.e. should not shut it off completely, it is a non-occlusive ligature), the arteriovenous fistula should be localized distally from the ligature, and there should be no branch (collateral vein) between the ligature and the fistula.

Since the above-described ligature and fistula authentically stimulate occurrence of PW syndrome with its characteristic symptoms, the model can be used to investigate the mechanisms of the limb hypertrophy and muscle, bone and connective tissue alterations during the development of PW syndrome. Further, creation of arteriovenous fistula and a ligature in young animals permits to study the mechanisms leading to both prenatal and postnatal malformations (fistulas in PW syndrome are congenital and certain abnormalities appear during the embryonic period). Further, the model according to the invention is also an effective research tool in pharmacological studies and studies on materials for vascular therapy.

The non-human animal mammalian that can be used for preparing the animal model is preferably a rodent, a lagomorph, a monkey and the like. Among rodents a mouse, a rat, a hamster, a guinea pig and the like can be used, preferably a rat. Preferably the animal is young and sexually mature. Male or female, body weight and the like characteristics of animals should be in accordance with generally accepted requirements for operations being performed in the preparation of the disease model.

Preferably, the ligature and the fistula are created in only one of the limbs, more preferably in one of the hind limbs, wherein the second hind limb preferably serves as a control limb.

The arteriovenous fistula is preferably situated on common femoral vessels below superior epigastric vessels. The size of the fistula depends on the size of the animal used, and it can be described e.g. by reference to longitudinal long incisions on common femoral vessels, having the length between 1 and 3 times of the diameter of the incised vein, preferably 1.5 and 2.5 times the diameter of the incised vein, most preferably around 2 times the diameter of the incised vein. The fistula can be created with use of standard, known materials. Suture is preferably non-absorbable, such as monofilament non-absorbable suture, of size, for example 10/0. Suture is preferably continuous, knots are situated outside the vessels. The arteriovenous fistula can by created by any conventional method, such as the fistula following needle puncture and gluing the vessels or anastomosis, preferably it is created by anastomosis.

The ligature is situated proximally to the arteriovenous fistula, preferably as close as possible, however, far enough not to disturb blood flow in anastomosis. There cannot be any vein branch between arteriovenous fistula and ligature - such as branch vein (collateral vein) as it would influence blood pressure and lead to blood steal. The ligature only narrows the vein lumen (i.e. should not shut it off completely), preferably the vein lumen after the intervention is about 50 to 100% of the initial vein lumen, more preferably 60% to 95% of the initial vein lumen, most preferably 70 to 90 % of the initial vein lumen. The ligature limits blood flow from common femoral vein. The ligature can be created with use of standard known materials. Suture is non-absorbable, such as monofilament suture, of a size for example 5/0, 6/0 or 7/0.

The present invention also provides a method for preparing the animal model. The method includes:
- anaesthetizing the animal,
- incising skin of at least one of the limbs,
- dissecting common femoral vessels from surrounding tissues, preferably proximally to the orifice of superior epigastric vessels,
- application of micro-vascular clamps on common femoral vessels, creating an arteriovenous fistula,
- removing microvascular clamps,
- application of a non-absorbable ligature on a common femoral vein, which limits blood flow from common femoral vein to inferior cava vein.

It is also possible to apply ligature first, and subsequently create an arteriovenous fistula, however, it is more difficult from a technical point of view.

An arteriovenous fistula can be created by any conventional method as long as the resultant fistula fulfills the requirements set out above, in particular is of the size required by the invention. It can be a fistula following needle puncture or fistula created by anastomosis, including side-to-side anastomosis (side of the vein to side of the artery), side-to-end anastomosis (side of the vein to end of the artery, or end of the vein to side of the artery), and end-to-end anastomosis (end of the vein to end of the artery). The inventors created the fistula by different methods and observed that the most preferred method is side-to-side anastomosis. This method allows the obtainment of reproducible results of the surgical intervention. This preferred method includes incising a common femoral vein and the artery, preferably on their adjacent sides, more preferably an antero-lateral side of the common femoral vein and preferably antero-medial side of the artery, and afterwards creating the arteriovenous fistula by side-to-side anastomosis between common femoral vessels.

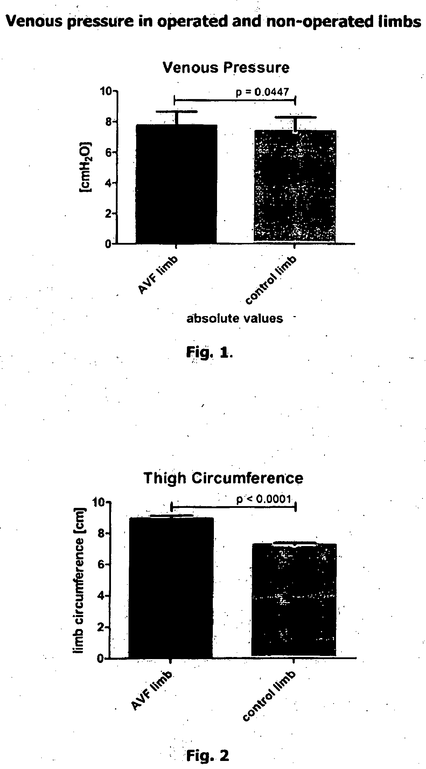

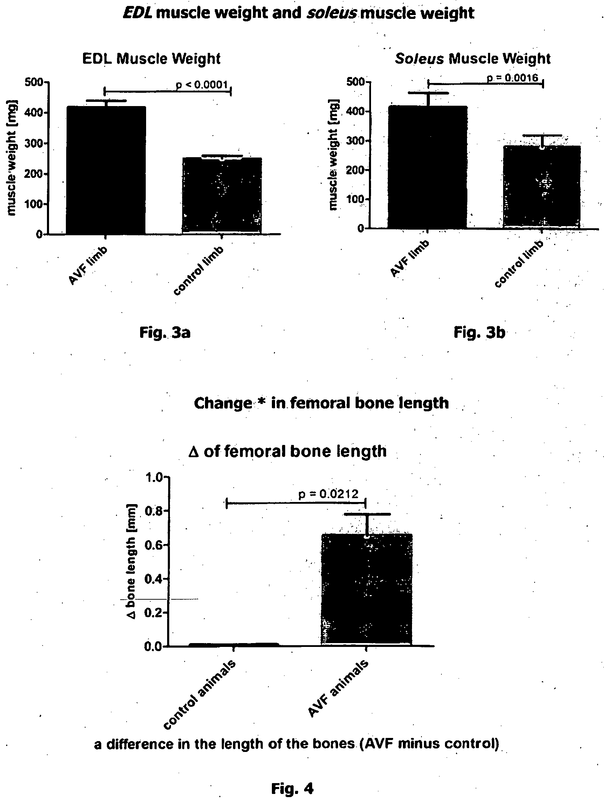

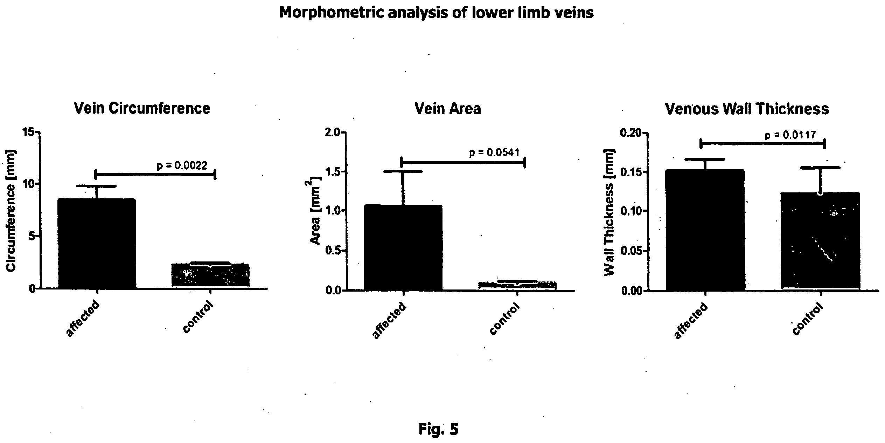

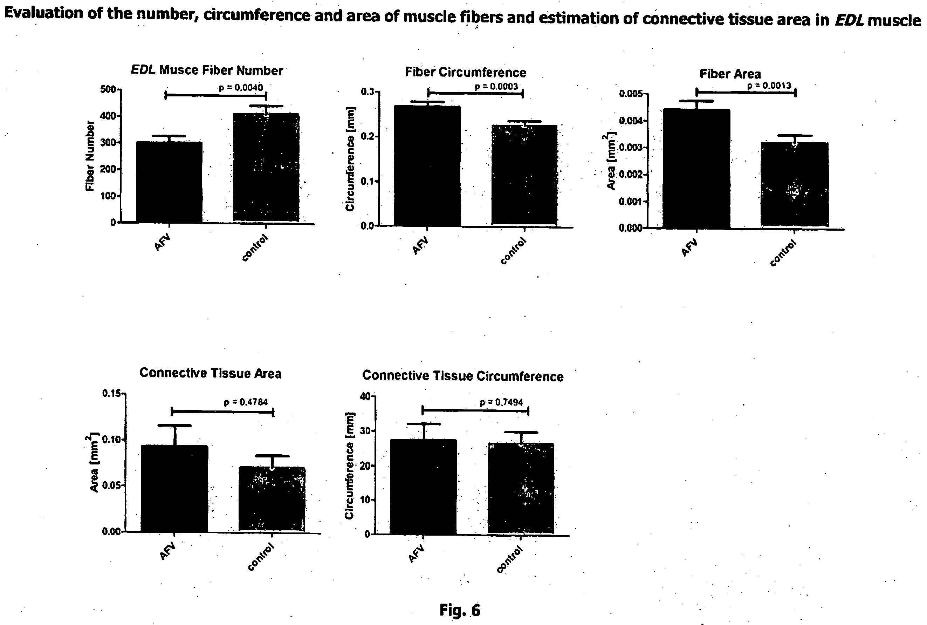

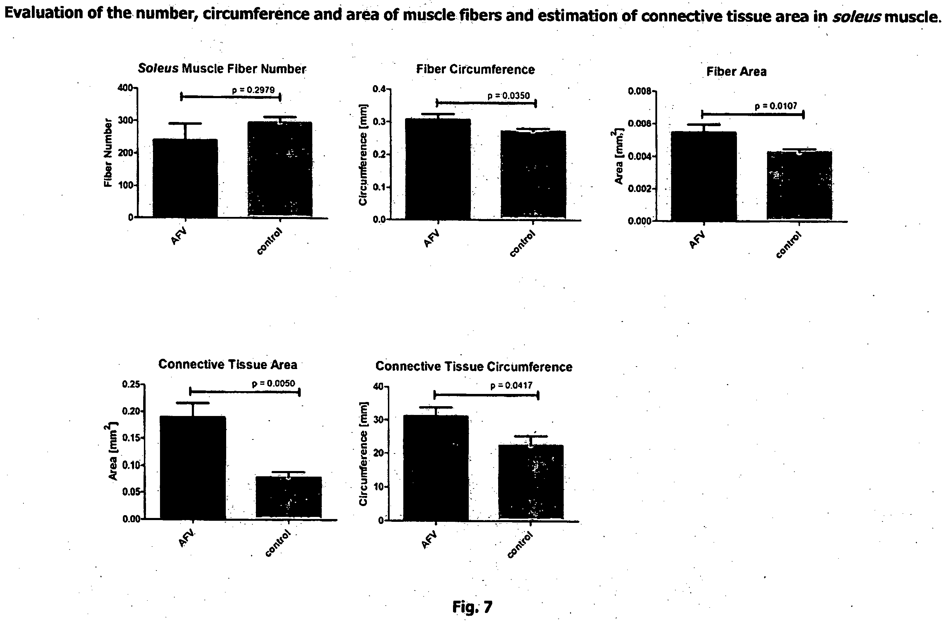

The surgical creation of the above-described arteriovenous fistula and the ligature leads to chronic venous insufficiency, and induces the overgrowth of a limb. The phenomenon is manifested by the increase of the limb circumference and the bone length and by muscle hypertrophy which is due to muscle regeneration and ischemia induced perivascular fibrosis. Several analyses have been performed in order to confirm the observation:
- measurements of venous pressure in operated and non-operated limbs (Fig. 1),
- thigh measurement (Fig. 2),
- weight assessment of *extensor digitorum longus* (*EDL*) and *soleus* muscles (Fig. 3a and 3b),
- measurement of the hind limb long bones (femoral bones) (Fig. 4),
- morphometric analysis of lower limb veins (Fig. 5),
- evaluation of the number, circumference and area of muscle fibers and estimation of connective tissue area in *EDL* muscle (Fig. 6),
- evaluation of the number, circumference and area of muscle fibers and estimation of connective tissue area in *soleus* muscle (Fig. 7).

PW syndrome is a particular example of venous insufficiency, and the presented data indicate that the inventors created an animal model of this disease. The unpredicted symptoms, including enlargement of a limb have been manifested in all operated animals.

The time period between the preparation of the model and occurrence of symptoms of PW syndrome depends on many factors, the most important of which are:
- size of arteriovenous fistula (determines blood flow through the fistula, arteries and veins, particularly localized distally from the fistula),
- vein lumen after application of the ligature, the ligature being situated proximally to arteriovenous ligature (determines flow rate and blood pressure distribution)
- age of the animal.

The inventors observed that histopathological alterations are constant processes. Certain alterations will be observed shortly after the operation, while others after a longer period; for example, it takes a considerably longer period to develop femoral bone elongation than alterations in veins and muscles, whereas venous insufficiency can be observed shortly after the operation.

The inventors observed that 6-10 weeks old rats, with fistula on common femoral veins of size corresponding to about 2 mm incisions, and proximal vein lumen limited by ligature to about 0.9 mm, developed symptoms of PW syndrome within around 30 days following the operation.

In the next aspect the invention relates to a method for studying Parkes Weber syndrome, wherein an animal model of the inventions is studied. The method preferably includes at least one of the following measurements: measuring vein pressure, measuring circumference of the limbs, measuring mass of the muscles, measuring length of the bones. The method may also include administering of a drug. After the measurements and optional drug administration, the animals are optionally sacrificed and further analysis, preferably histopathologic one, is performed.

The animal model can be used for studying PW syndrome, comprising thorough assessment of animal physiology *in vivo*, such as invasive/non-invasive blood pressure measurements and oxygen saturation and *ex vivo* examination of animal tissues and body fluids.

The animal model for PW syndrome can also be used for screening potential materials for use in therapies, including materials for vascular use, such as stents.

The invention also provides a method of screening a preventive and/or therapeutic agent for Parkes Weber syndrome. In this method a test substance is administered to the animal model according to the invention and further studies are performed on the animal.

### EXAMPLES

### Example 1

### Method of preparing an animal model for PW

### Materials

The experiment was performed on male Wistar rats, at the age of 6 to 10 weeks, divided into two groups: the experimental group (n=14) and control group (n=3). The experiment was approved by the local Bioethics Commission for experiments on animals, Warsaw 02-091, Zwirki i Wigury 61 (resolution nr 46/2012).

### Method

### Animal model of PW syndrome - technique description

After induction of general anesthesia longitudinal skin incision just below inguinal ligament was performed. Common femoral vessels were dissected from surrounding tissues proximally to the orifice of superior epigastric vessels. After application of micro-vascular clamps on common femoral vessels longitudinal 2mm ± 0,1 mm long incision on antero-lateral side of the vein and antero-medial side of the artery were performed. Afterwards side-to-side anastomosis between common femoral vessels was performed to create arteriovenous fistula (non-absorbable monofilament suture 10/0). Subsequently micro-vascular clamps were removed and arteriovenous fistula patency was checked and haemostasis was achieved. Blood flow from common femoral vein to inferior cava vein was limited by application of non-absorbable ligature of 0.9 mm diameter (the resultant vein lumen corresponds to about 80-90% of the initial vein lumen). Next subcutaneous tissue and skin were sutured with absorbable suture.

Control limb operation included skin incision, common femoral vessels dissection, and subcutaneous and skin suture (absorbable suture).

### Example 2

### Blood pressure measurement

The animal models were prepared according to the method described in example 1.

In anesthetized control and studied rats (median observation time after arteriovenous fistula creation - 30 days) invasive blood pressure measurement in inferior cava vein and both common iliac veins - one with arteriovenous fistula, and without arteriovenous fistula - were performed. Invasive venous pressure was measured with Millar pressure transducer and PowerLab monitoring and acquisition system (ADInstrumehts, UK). Data were collected and analyzed with LabChart software (ADInstruments, UK). Venous pressure was presented as centimeters of water (cmH₂O).

### Statistical analysis

The results were expressed as arithmetic means and standard errors of the mean (SEM). Comparisons between the results obtained for the examined limbs and the results for control limbs were performed using the paired t-test (GraphPad Prism, v.6.01). The differences at p < 0.05 were considered statistically significant.

Fig. 1 shows the results of measurements of venous pressure in operated and non-operated limbs (mean venous pressures of 7.76 and 7.41 cmH₂O in the operated and control limbs, respectively; p=0.04).

### Example 3

### Tigh circumference, muscle weight, femoral bone length measurements

The animal models were prepared according to the method described in example 1.

After the euthanasia measurements of tigh circumference of arteriovenous and control limbs were performed. *Soleus* muscle and the extensor digitorum longum (EDL) muscle were dissected from posterior part of hind limb, excised and weight. Femoral bones were excised and their length was measured with electronic slide clliper.

### Statistical analysis

The results were expressed as arithmetic means and standard errors of the mean (SEM). Comparisons between the results obtained for the examined limbs and the results for control limbs were performed using the paired t-test (GraphPad Prism v.6.01). The differences at p < 0.05 were considered statistically significant.
Fig. 2 shows results of measurements of thigh circumference of operated and non-operated limb
Fig. 3a and 3b show results of measurements of *EDL* muscle weight and *soleus* muscle weight
Figure 4 shows change * in femoral bone length.
   *The first bar depicts the difference in bone length in non-operated animals (i.e. the length of the right femoral bone subtracted from the left femoral bone). The second bar represents changes in bone length in operated animals (i.e. the length of femoral bone from the right, non-operated limb subtracted from the length of femoral bone of the left, operated limb).

The raw data of limb circumference, EDL and *soleus* muscle weights as well as of the differences in bone length [mm] measurements are listed in Tab. 1.

The mean values of these measurements in the AVF limbs compared to the control limbs were as follows:
- limb circumference: 8.94 *vs* 7.26 cm,
- *EDL* muscle weight: 418.23 *vs* 250.64 mg,
- *Soleus* muscle weight: 416.50 *vs* 281.86 mg, and
- Δ of bone length: 0.65 *vs* 0.01 mm.

**Tab. 1.**

| **limb circumference [cm]** | | ***EDL* muscle weight [mg]** | | ***Soleus* muscle weight [mg]** | | **Δ of bone length [mm]** | |
|---|---|---|---|---|---|---|---|
| **AVF limb** | **control limb** | **AVF limb** | **control limb** | **AVF limb** | **control limb** | **AVF animals** | **control animals** |
| 7,2 | 6,5 | | 203 | 259 | 206 | 1,26 | 0,01 |
| 8,3 | 7,01 | 414 | 240 | 507 | 280 | 0,65 | 0,01 |
| 8,8 | 7,5 | 388 | 280 | 515 | 337 | 0,49 | 0,01 |
| 8,8 | 7,5 | 444 | 230 | 834 | 750 | 0,39 | |
| 8,6 | 7,2 | 327 | 240 | 302 | 207 | 0,05 | |
| 8,9 | 6,7 | 376 | 226 | 295 | 197 | 0,16 | |
| 8,4 | 7 | 418 | 242 | 347 | 234 | 0,85 | |
| 8,5 | 7 | 322 | 227 | 286 | 204 | 1,11 | |
| 9,4 | 7,2 | 462 | 283 | 266 | 251 | 0,42 | |
| 9,5 | 7,2 | 426 | 246 | 346 | 206 | 1,2 | |
| 9 | 7,4 | 302 | 239 | 754 | 218 | 0,62 | |
| 9,9 | 8,1 | 558 | 327 | 407 | 274 | | |
| 10,1 | 7,9 | 467 | 263 | 398 | 340 | | |
| 9,7 | 7,4 | 533 | 263 | 315 | 242 | | |

### Example 4

### The histopathologic analysis of the venous vessels as well as soleus and extensor digitorum longus muscles

The animal models were prepared according to the method described in example 1.

Following the euthanasia of the animals, the fragments of common femoral veins at the site where the arteriovenous fistulas were created (common femoral vein, common artery) as well as *soleus* and *extensor digitorum longus* muscles were excised and frozen by placing them for several seconds in 2-methylbutane (Sigma-Aldrich, USA) cooled in liquid nitrogen. Then the samples were transferred to liquid nitrogen and stored at - 80°C until the preparation of histological slides. Tissues were cut using a cryostat into 10µm-thick sections and mounted on the glass slides for 30 minutes in 37°C allowing them to dry (slides with mounted cryosections can be stored at 4°C). Next, the slides were placed in phosphate buffered saline (PBS) for 15 minutes and then stained for 40 minutes with Harris's hematoxylin (Sigma-Aldrich, USA). After washing with tap water, the slides were stained with eosin Y (Sigma-Aldrich, USA) for 3 minutes. Specimen were again washed with tap water and then mounted using UltraMount (DakoCytomation, Denmark). The examined sections were viewed under the light microscope (Nikon Eclipse TE200; Nikon Instruments, Inc., Japan) under the 4x magnification lens and photographed using the NIS Elements F 2.30 software (Nikon Instruments, Inc., Japan) at the same resolution (2560x1920 pixels).

Morphometric analyses of the sections were performed using the Adobe Photoshop CS5 Extended software (version 12.0 x32). Fiber numbers within individual muscles, areas and circumferences of the veins as well as venous wall thickness and the amount of connective tissue were assessed. Additionally, the venous wall thickness was measured at three different sites - at the position of the lowest, mean (the most common) and the highest thickness. The measuring tool was used and the mean value of the venous wall thickness was calculated.

### Statistical analysis

The results were expressed as arithmetic means and standard errors of the mean (SEM). Comparisons between the results obtained for the examined limbs and the results for control limbs were performed using the paired t-test (GraphPad Prism v.6.01). The differences at p < 0.05 were considered statistically significant.

Figure 5 shows results of morphometric analysis in lower limb veins.

Figure 6 shows evaluation of the number, circumference and area of muscle fibers and estimation of connective tissue area in *EDL* muscle.

Figure 7 shows evaluation of the number, circumference and area of muscle fibers and estimation of connective tissue area in *soleus* muscle.

The raw data resulting from the morphometric analysis of lower limb veins are listed in Tab. 2a. The mean values of the measurements in the affected veins compared to the control veins were as follows:
- Vein Circumference: 8.43 *vs* 2.29 mm,
- Vein Area: 1.06 *vs* 0.10 mm², and
- Venous Wall Thickness: 0.15 *vs* 0.12 mm.

The raw data resulting from the morphometric analysis of *EDL* and *soleus* muscles are listed in Tab. 2b. The mean values of the measurements in the *EDL* muscles in AVF limbs compared to the control limbs were as follows:
- Muscle Fiber Number: 301.27 *vs* 411.67,
- Fiber Circumference: 0.27 *vs* 0.23 mm,
- Fiber Area: 0.0044 *vs* 0.0032 mm²,
- Connective Tissue Area: 0.0936 *vs* 0.0717 mm²,
- Connective Tissue Circumference: 27.39 *vs* 26.73 mm,

The mean values of the measurements in the *soleus* muscles in AVF limbs compared to the control limbs were as follows:
- Muscle Fiber Number: 240.58 *vs* 295.83,
- Fiber Circumference: 0.31 *vs* 0.27 mm,
- Fiber Area: 0.0055 *vs* 0.0043 mm²,
- Connective Tissue Area: 0.19 *vs* 0.08 mm²,
- Connective Tissue Circumference: 31.19 *vs* 22.57 mm.

**Tab. 2a.**

| **Morphometric analysis of lower limb veins** | | | | | |
|---|---|---|---|---|---|
| **Vein Circumference [mm]** | | **Vein Area [mm²]** | | **Venous Wall Thickness [mm]** | |
| **affected** | **control** | **affected** | **control** | **affected** | **Control** |
| 4.60 | 1.80 | 0.10 | 0.01 | 0.14 | 0.06 |
| 16.14 | 2.13 | 4.29 | 0.13 | 0.21 | 0.08 |
| 7.00 | 2.81 | 1.61 | 0.21 | 0.18 | 0.09 |
| 1.56 | | 0.01 | | 0.07 | |
| 11.66 | 1.86 | 1.33 | 0.12 | 0.11 | 0.12 |
| 8.09 | 1.92 | 0.90 | 0.08 | 0.12 | 0.11 |
| 9.24 | 2.26 | 0.72 | 0.04 | 0.17 | 0.10 |
| | 2.24 | | 0.21 | | 0.07 |
| | 3.38 | | 0.07 | | 0.38 |
| 8.80 | 2.24 | 0.32 | 0.02 | 0.17 | 0.09 |
| 8.80 | | 0.27 | | 0.20 | |

**Tab. 2b.**

| **Morphometric analysis of *EDL* muscles** | | | | | | | | | | **Morphometric analysis of *soleus* muscles** | | | | | | | | | |
|---|---|---|---|---|---|---|---|---|---|---|---|---|---|---|---|---|---|---|---|
| **Muscle Fiber Number** | | **Fiber Circumference [mm]** | | **Fiber Area [mm²]** | | **Connective Tissue Area [mm²]** | | **Connective Tissue Circumference [mm]** | | **Muscle Fiber Number** | | **Fiber Circumference [mm]** | | **Fiber Area [mm²]** | | **Connective Tissue Area [mm²]** | | **Connective Tissue Circumference [mm]** | |
| **AFV** | **control** | **AFV** | **control** | **AFV** | **control** | **AFV** | **control** | **AFV** | **control** | **AFV** | **control** | **AFV** | **control** | **AFV** | **control** | **AFV** | **control** | **AFV** | **control** |
| 354 | 431 | 0.24 | 0.20 | 0.0035 | 0.0030 | 0.0677 | 0.0398 | 26.65 | 19.46 | 217 | 292 | 0.3057 | 0.2781 | 0.0056 | 0.0042 | 0.30 | 0.10 | 48.60 | 34.17 |
| 278 | 570 | 0.23 | 0.20 | 0.0033 | 0.0024 | 0.2900 | 0.0224 | 60.91 | 13.84 | 784 | 304 | 0.1539 | 0.2577 | 0.0015 | 0.0039 | 0.10 | 0.13 | 39.65 | 17.78 |
| 271 | 498 | 0.27 | 0.20 | 0.0045 | 0.0024 | 0.0543 | 0.1190 | 17.68 | 39.23 | 124 | 216 | 0.3583 | 0.3131 | 0.0072 | 0.0056 | 0.27 | 0.07 | 24.17 | 39.71 |
| 198 | 378 | 0.32 | 0.23 | 0.0061 | 0.0033 | 0.0874 | 0.1002 | 22.17 | 30.17 | 195 | 272 | 0.3354 | 0.2807 | 0.0065 | 0.0044 | 0.11 | 0.14 | 17.61 | 34.69 |
| 376 | 440 | 0.24 | 0.21 | 0.0034 | 0.0027 | 0.0522 | 0.0613 | 20.95 | 30.60 | 202 | 286 | 0.3032 | 0.2764 | 0.0051 | 0.0044 | 0.23 | 0.08 | 33.77 | 17.91 |
| 377 | | 0.24 | | 0.0035 | | 0.0365 | | 10.57 | | 257 | 445 | 0.2705 | 0.2184 | 0.0041 | 0.0028 | 0.26 | 0.08 | 17.77 | 14.00 |
| 446 | | 0.22 | | 0.0029 | | 0.0520 | | 19.86 | | 196 | 365 | 0.3140 | 0.2440 | 0.0057 | 0.0034 | 0.13 | 0.06 | 29.06 | 19.09 |
| 224 | 332 | 0.29 | 0.24 | 0.0052 | 0.0037 | 0.0859 | 0.1197 | 20.38 | 28.37 | 251 | 274 | 0.2862 | 0.2729 | 0.0045 | 0.0043 | 0.06 | 0.13 | 36.54 | 31.36 |
| 361 | 364 | 0.29 | 0.24 | 0.0049 | 0.0034 | 0.1403 | 0.0904 | 53.67 | 41.26 | 168 | 283 | 0.3329 | 0.2773 | 0.0061 | 0.0043 | 0.23 | 0.05 | 39.37 | 18.21 |
| 231 | 445 | 0.31 | 0.22 | 0.0055 | 0.0029 | 0.0347 | 0.0413 | 18.07 | 17.89 | 171 | 305 | 0.3167 | 0.2763 | 0.0058 | 0.0042 | 0.12 | 0.03 | 22.79 | 13.05 |
| | 247 | | 0.30 | | 0.0051 | | 0.0513 | | 19.74 | 210 | 260 | 0.3225 | 0.2951 | 0.0056 | 0.0049 | 0.12 | 0.03 | 29.61 | 13.76 |
| 198 | | 0.30 | | 0.0057 | | 0.1281 | | 30.39 | | 112 | 248 | 0.3873 | 0.2894 | 0.0079 | 0.0049 | 0.35 | 0.05 | 35.31 | 17.16 |

### Example 5

### The implementation of the established model - a method for screening for a therapeutic agent for PW syndrome

### A research study on β-escin, as a new drug in the PW syndrome treatment

The animal models were prepared according to the method described in example 1.

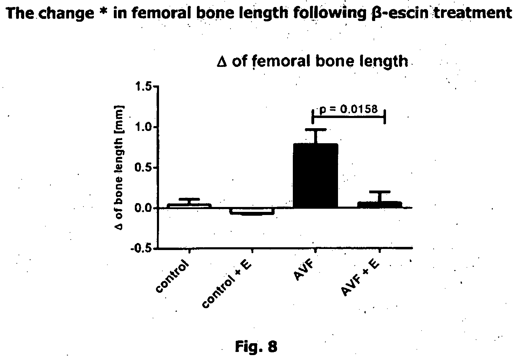

The animals with surgically created arteriovenous fistulas (AVF) were treated for 6 weeks with β-escin ("E", 30 mg of β-escin sodium salt administered by oral gavage). The measurements of the femoral bones revealed that β-escin significantly reduced the bone elongation which is characteristic for PW syndrome (Fig. 8)

**Tab. 3.**

| **Δ of femoral bone length [mm]** | | | |
|---|---|---|---|
| **control** | **control + β-escin** | **AVF** | **AVF + β-escin** |
| -0.12 | -0.06 | 0.6 | -0.21 |
| 0.35 | -0.08 | 2.75 | 0.71 |
| -0_{:}01 | | 0.45 | -0.26 |
| 0.01 | | 1.26 | 0.19 |
| 0.01 | | 0.65 | 0.04 |
| 0.01 | | 0.49 | -0.24 |
| | | 0.39 | 0.21 |
| | | 0.05 | |
| | | 0.16 | |
| | | 0.85 | |
| | | 1.11 | |
| | | 0.42 | |
| | | 1.2 | |
| | | 0.62 | |

**Fig. 8** **shows** the Change * in femoral bone length following β-escin treatment.
*The first two bars depicts the difference in bone length in non-operated (control) animals untreated and treated with β-escin (E) (i.e. the length of the right femoral bone subtracted from the left femoral bone). The following two bars represents changes in bone length in operated animals (AVF) which were untreated and treated with β-escin (E) (i.e. the length of femoral bone from the right, non-operated limb subtracted from the length of femoral bone of the left, operated limb).

The raw data resulting from this analysis are presented in Tab. 3. The mean values of the differences of bone lengths in the control animals were -0.07 and 0.04 mm with and without β-escin treatment, respectively. The differences in bone lengths in AVF animals were much more pronounced with mean values of 0.06 and 0.79 mm for rats undergoing and not subjected to β-escin treatment, respectively.

## Claims

1. A non-human animal mammalian model for Parkes Weber syndrome, wherein an animal has an arteriovenous fistula and a venous ligature in at least one of the limbs, the ligature narrows the vein lumen, and does not shut it off completely, the arteriovenous fistula is localized distally from the ligature, and there is no branch (collateral vein) between the ligature and the fistula.

2. The non-human animal mammalian model according to claim 1, wherein the animal is a rodent, preferably a rat.

3. The non-human animal mammalian model according to any of the previous claims, wherein the arteriovenous fistula and the ligature are localized in at least one of the hind limbs.

4. The non-human animal mammalian model according to any of the previous claims, wherein the size of the fistula corresponds to longitudinal long incisions on common femoral vessels having a length between 1 and 3 times a diameter of the incised vein, preferably 1.5 and 2.5 times a diameter of the incised vein, most preferably around 2 times a diameter of the incised vein.

5. The non-human animal mammalian model according to any of the previous claims, wherein the ligature narrows the vein lumen to between about 50% and about 100% of the initial vein lumen, more preferably 60% to 95% of the initial vein lumen, most preferably 70 to 90 % of the initial vein lumen.

6. A method for preparing the non-human animal mammalian model for Parkes Weber syndrome of claims 1-5, which comprises creating an arteriovenous fistula and making a non-occlusive ligature of a vein in at least one of the limbs of the animal, whereby the arteriovenous fistula is localized distally from the ligature, and there is no branch (collateral vein) between the ligature and the fistula.

7. A method for studying Parkes Weber syndrome, comprising studying the non-human animal mammalian model of claims 1-5.

8. The method according to claim 7, which comprises:
- providing the animal model as defined in claims 1-5,
- performing at least one of the following measurements: measuring vein pressure, measuring circumference of the limbs, measuring mass of the muscles, measuring length of the bones,
- sacrificing the animal, and
- performing further analysis, preferably histopathologic analysis.

9. The method according to claim 7 or 8 wherein the animal is administered a drug.

10. Use of the animal model as defined in claims 1-5 for studying Parkes Weber syndrome.

11. Use according to claim 10, wherein the studying comprises invasive/non-invasive blood pressure measurements in the concerned limb(s) and/or the organism of the animal.

12. Use of the animal model of claims 1-5 for screening a material for use in vascular therapy for Parkes Weber syndrome, such as stents.

13. A method of screening a preventive and/or therapeutic agent for Parkes Weber syndrome, wherein a test substance is administered to the animal model of claims 1-5.

## Patentansprüche

1. Nicht-humanes Säugetier-Modell für Parkes Weber Syndrom, bei dem bei einem Tier an mindestens einer Extremität eine arteriovenöse Fistel und eine Venenligatur angelegt wurden, die Ligatur verringert das Lumen des Blutgefäßes, verschließt es jedoch nicht vollständig, die arteriovenöse Fistel liegt distal zu der Ligatur und es besteht keine Verzweigung (kein Kollateralblutgefäß) zwischen der Ligatur und der Fistel.

2. Nicht-humanes Säugetiermodell gemäß Anspruch 1, bei dem das Tier ein Nagetier ist, vorzugsweise eine Ratte.

3. Nicht-humanes Säugetier-Modell gemäß allen vorhergehenden Ansprüchen, bei dem sich eine arteriovenöse Fistel und eine Ligatur mindestens an einer der Hinterextremitäten befinden.

4. Nicht-humanes Säugetier-Modell gemäß allen vorhergehenden Ansprüchen, bei dem die Fistelgröße den langen längs vorgenommenem Inzisionen an den gemeinsamen Oberschenkelgefäßen entspricht, deren Länge zwischen dem einfachen und dem dreifachen Durchmesser des Blutgefäßes liegt, an dem die Inzision vorgenommen wurde, wobei die Länge vorzugsweise zwischen dem eineinhalbfachen und dem zweifachen Durchmesser des Blutgefäßes liegen soll, an dem die Inzision vorgenommen wurde, ganz besonders bevorzugt wenn sie ungefähr dem zweifachen Durchmesser des Blutgefäßes entspricht, an dem die Inzision vorgenommen wurde.

5. Nicht-humanes Säugetier-Modell gemäß allen vorhergehenden Ansprüchen, bei dem die Ligatur das Gefäßlumen von ca. 50% auf ca. 100% des ursprünglichen Gefäsßlumens verringert, vorzugsweise zwischen 60% und 95% des ursprünglichen Gefäßlumens, ganz besonders bevorzugt zwischen 70% und 90% des ursprünglichen Gefäßlumens.

6. Verfahren zur Vorbereitung eines nicht-humanen Säugetiermodells für das Parkes Weber Syndrom zur Verwendung gemäß Ansprüchen 1-5, welches aus dem Anlegen einer arteriovenösen Fistel und einer nicht-okklusiven (den Blutfluss nicht verschließenden) Gefäßligatur an mindestens einer Extremität des Tieres besteht, wodurch die arteriovenöse Fistel distal zu der Ligatur angeordnet wird und es zwischen der Ligatur und der Fistel keine Verzweigung (kein Kollateralblutgefäß) gibt.

7. Verfahren zur Untersuchung des Parkes Weber Syndroms gemäß Ansprüchen 1-5, bestehend aus der Untersuchung des nicht-humanen Säugetier-Modells.

8. Verfahren gemäß Anspruch 7, bestehend aus:
- Zurverfügungstellung eines nicht-humanen Säugetier-Modells gemäß der Beschreibung nach Ansprüchen 1-5,
- Vornahme mindestens einer der folgenden Messungen: Messung des intravenösen Drucks, Messung des Umfangs der Extremitäten, Messung der Muskelmasse, Messung der Knochenlänge,
- Einschläferung des Tieres und
- Vornahme von weiteren Untersuchungen, wobei eine histopahologische Untersuchung bevorzugt wird.

9. Verfahren gemäß Anspruch 7 oder 8, bei dem die Verabreichung eines Arzneimittels an das Tier vorgesehen ist.

10. Verwendung eines nicht-humanen Säugetiermodells gemäß der Beschreibung gemäß Ansprüchen 1-5 zwecks Vornahme der Studien am Parkes Weber Syndrom.

11. Verwendung gemäß Anspruch 10, bei dem invasive / nicht-invasive Blutdruckmessungen an bestimmten Extremitäten / einer bestimmten Extremität und/oder im Körper des Tieres vorgenommen werden.

12. Verwendung eines nicht-humanen Tier-Modells gemäß Ansprüchen 1-5 zur Prüfung nach einem Screening-Verfahren des bei der Gefäßtherapie bei vorliegendem Parkes Weber Syndrom eingesetzten Materials, wie Stents.

13. Screening-Verfahren zur Untersuchung eines bei dem Parkes Weber Syndrom prophylaktisch und/oder therapeutisch zu verabreichenden Mittels, bei dem das zu untersuchende Mittel an ein nicht-humanes Tier-Modell gemäß der Beschreibung gemäß Ansprüchen 1-5 verabreicht wird.

## Revendications

1. Un modèle animal, non-humain, mammifère, du syndrome de Parkes Weber où des animaux présentent une fistule artério-veineuse et une ligature de veine sur au moins un membre du corps où la ligature rétrécit la lumière de la veine, mais ne l'obture pas complètement et la fistule artério-veineuse est située distalement par rapport à la ligature et où il n'y a pas de branche collatérale entre la ligature et la fistule.

2. Un modèle animal, non-humain, mammifère, selon la revendication 1 où l'animal est un rongeur, de préférence un rat.

3. Un modèle animal, non-humain, mammifère, selon les revendications précédentes où la fistule artério-veineuse et la ligature de veine sont situées au moins sur une patte arrière.

4. Un modèle animal, non-humain, mammifère, selon les revendications précédentes où la taille de la fistule correspond à de longues incisions longitudinales des vaisseaux fémoraux communs dont la longueur est égale de 1 à 3 diamètres de la veine incise où de préférence avec la longueur égale de 1,5 à 2,5 diamètres de la veine, au mieux à 2 diamètres de la veine incise.

5. Un modèle animal, non-humain, mammifère, selon les revendications précédentes où la ligature de veine rétrécit la lumière de la veine entre environ 50% à environ 100% de la lumière initiale de la veine, de préférence de 60% à 95% de la lumière initiale de la veine, au mieux de 70 % à 90 % de la lumière initiale de la veine.

6. Une méthode de préparation d'un modèle animal, non-humain, mammifère, du syndrome de Parkes Weber selon les revendications 1 à 5 qui comprend la création d'une fistule artério-veineuse et une ligature de veine non-occlusive sur au moins un membre du corps de l'animal ce qui place la fistule artério-veineuse distalement par rapport à la ligature de veine et ne forme pas de branche, (une veine collatérale) entre la ligature et la fistule.

7. Une méthode d'examen du syndrome de Parkes Weber qui comprend l'examen du modèle animal, non-humain, mammifère, selon les revendications 1 à 5.

8. Une méthode selon la revendication 7 qui comprend :
- la fourniture d'un modèle animal décrit dans les revendications 1 à 5,
- la réalisation d'au moins un examen parmi les examens suivants : mesure de la tension artérielle, mesure de la circonférence des membres du corps, mesure de la masse musculaire, mesure de la longueur des os,
- la mise à mort de l'animal, et
- la poursuite des analyses, de préférence une analyse histopathologique.

9. Une méthode selon la revendication 7 ou 8 qui prévoit l'administration d'un médicament à l'animal.

10. L'utilisation d'un modèle animal décrit dans les revendications 1 à 5, pour examiner le syndrome de Parkes Weber.

11. L'utilisation selon la revendication 10 qui prévoit des mesures invasives / non-invasives de la tension artérielle dans les membres du corps concernés / le membre du corps concerné et/ou dans l'organisme de l'animal.

12. L'utilisation du modèle animal selon les revendications 1 à 5 pour l'examen, par la voie de dépistage, du dispositif utilisé pour le traitement des veines dans le cas du syndrome Parkes Weber, d'un stent par exemple.

13. Une méthode de dépistage pour examiner un moyen préventif et/ou thérapeutique du syndrome de Parkes Weber où la substance analysée est introduite au modèle animal selon les revendications 1 à 5.
